# EUROPÄISCHE PATENTSCHRIFT

(11) **EP 2 319 566 B1**
(45) Veröffentlichungstag und Bekanntmachung des Hinweises auf die Patenterteilung: **11.07.2012**
(21) Anmeldenummer: 10014210.8
(22) Anmeldetag: 02.11.2010
(51) Int. Cl.: A61M 13/00, A61F 9/007

(54) **Spritze zur Injektion eines chirurgischen Gases**
Syringe for injecting a surgical gas
Seringue pour injection d'un gas chirugical

(30) Priorität: 10.11.2009 DE 102009052552
(43) Veröffentlichungstag der Anmeldung: 11.05.2011
(73) Patentinhaber: FLUORON GMBH, 89077 Ulm (DE)
(72) Erfinder: Lingenfelder, Christian, Dr., 89081 Ulm (DE); Knopp, Benjamin, 89134 Blaustein (DE)
(74) Vertreter: Schiweck, Weinzierl & Koch

(56) Entgegenhaltungen:
- EP-A1- 2 020 245
- FR-A1- 2 850 564
- US-A- 6 073 759
- US-B1- 6 599 280

## Beschreibung

Die Erfindung betrifft eine Spritze gemäß dem Oberbegriff des Anspruches 1.

Eine derartige Spritze ist aus der EP 2 020 245 A1 bekannt.

Insbesondere betrifft die Erfindung ein Ready to Use-System einer Gastamponade zur Anwendung in der Medizin. Die Tamponade wird zum Auffüllen einer natürlichen, z.B. durch eine alterungsbedingte Glaskörperschrumpfung, oder künstlichen, z.B. durch eine Vitrektomie entstandenen Kavität verwendet.

### Hintergrund der Erfindung

Bedingt durch die steigende Lebenserwartung des Menschen nehmen alterungsbedingte Erkrankungen wie Netzhautveränderungen bzw. Netzhautablösungen, grüner Star, grauer Star, sowie alterungsbedingte Makuladegeneration und diabetesbedingte Retinopathie zu. Um diese und auch weitere Erkrankungen des Auges zu behandeln, ist meistens eine Vitrektomie (Entfernung des Glaskörpers) notwendig. Der entstandene Hohlraum muss wieder aufgefüllt werden, um ein Zusammenfallen des Glaskörperraumes zu verhindern. Zu diesem Zweck werden "schwere Gase" wie SF₆, C₂F₆ oder C₃F₈ verwendet. Von schweren Gasen spricht man allgemein, wenn die Gase eine deutlich höhere Dichte im Vergleich zu normaler Raumluft besitzen.

Die therapeutische Wirkung entsteht in den meisten Fällen nicht durch das Gas selbst, sondern vielmehr durch die Gas-Flüssigkeitsgrenzfläche. Diese Oberflächenspannung beugt dem Durchtritt von Gas durch ein Netzhautloch in den Subretinalraum vor, zusätzlich wird das Netzhautloch ausgespannt und ein weiteres Durchtreten von Flüssigkeit in den Subretinalraum verhindert.

Nach der Injektion des schweren Gases beginnt eine Diffusion von O₂ und CO₂ aus dem Blut ins Auge, wodurch das Volumen der Gasblase zunimmt. Nach einigen Stunden stellt sich ein Diffusionsgleichgewicht für O₂ und CO₂ ein, für N₂ jedoch erst nach einigen Tagen.

Die schweren Gase werden transretinal über die Aderhaut aufgenommen und verlassen je nach Typ das Auge innerhalb von 1 bis 2 Wochen.

Die in der Chirurgie verwendeten Gase werden in den meisten Fällen direkt während der Operation aus einer Stahlflasche in das zu verwendende Medium überführt.

Das US-Patent 6,866,142 B2 oder 6 073 759 A beschreibt ein System, welches aus einer bereits gasgefüllten Einmalspritze besteht, die zur besseren Gasdichtigkeit in einem Behälter aufbewahrt wird, der mit dem gleichen Gas wie die Einmalspritze gefüllt ist.

Die US 6 599 280 B1 und die FR 2 020 245 A beschreiben jeweils chirurgischen Kit mit einem separaten Gasspeicher, einer Spritze und weiteren mit der Spritze kombinierbaren Komponenten, die in einer Verpackung untergebiochet.

Alle oben aufgeführten Systeme haben mehrere Nachteile.
- Bei allen Systemen werden zum Teil erhebliche Mengen an SF₆, C₂F₆ und C₃F₈ in die Umwelt entlassen, obwohl diese Gase zu den stärksten bekannten Treibhausgasen zählen; so hat z.B. 1 kg SF₆ die gleichen Auswirkungen wie 22,2 t CO₂ [Verordnung EG Nr. 842/2006].
- Die Abfüllung von Gastamponaden aus der Stahlflasche im OP ist nicht zugelassen, da Stahlflaschen in einem speziellen Stahlschrank für Druckbehälter aufbewahrt werden müssen.
- Die auf dem Markt befindlichen Einmaldosen müssen in mehreren Schritten vorbereitet werden, bevor sie vom Chirurgen verwendet werden können.

Es ist daher Aufgabe der vorliegenden Erfindung, eine Spritze der im Oberbegriff des Anspruchs 1 angegebenen Art zu schaffen, die dazu in der Lage ist, die zuvor erläuterten Nachteile des Standes der Technik auszuräumen und insbesondere einfach aufzubewahren und zu bedienen ist.

Die Lösung dieser Aufgabe erfolgt durch die Merkmale des Anspruchs 1.

Die Unteransprüche haben vorteilhafte Weiterbildungen der Erfindung zum Inhalt.

### Detaillierte Beschreibung der Erfindung

Somit betrifft die Erfindung ein System mit einer Einmalspritze, vorzugsweise 50 ml, mit Luer-Lock-Ansatz, welche als Gasbehälter dient, mit einem auf die Spritze aufgeschraubten Spritzenvorsatzfilter mit beiderseitigem Luer-Lock-Anschluss und mit einem Luer-Stopfen, um das System zu verschließen.

Das montierte System (Einmalspritze und Spritzenvorsatzfilter) wird mit wenigen Millilitern des jeweiligen Gases gefüllt und mit dem Luer-Stopfen verschlossen.

Das System wird vorzugsweise mit einem Aufkleber versehen, welcher die Mischungsverhältnisse für SF₆ (20% Gas/80% Luft), C₂F₆ (16% Gas/84% Luft) und C₃F₈ (12% Gas/88% Luft) farblich unterscheidet, sowie den Maximumwert kennzeichnet, bis zu dem der Kolben herausgezogen werden muss/darf, um das Mischungsverhältnis einzustellen. Das verschlossene System wird verpackt, vorzugsweise in einen Sterilbeutel, und anschließend sterilisiert, vorzugsweise in einem Dampfautoklaven.

Weitere Einzelheiten, Merkmale und Vorteile der Erfindung ergeben sich aus nachfolgender Beschreibung von Ausführungsbeispielen anhand der Zeichnung.

Darin zeigt:
Fig. 1 eine schematisch leicht vereinfachte Darstellung einer ersten Ausführungsform einer erfindungsgemäßen Spritze, und
Fig. 2 eine der Fig. 1 entsprechende Darstellung einer zweiten Ausführungsform der erfindungsgemäßen Spritze.

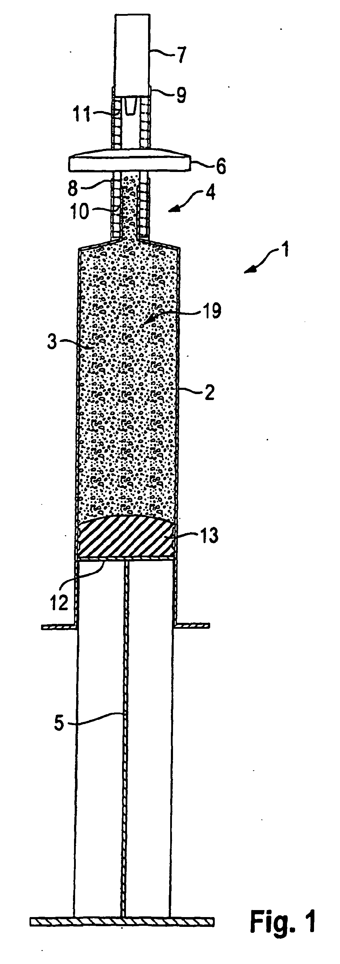

In Fig. 1 ist eine erste Ausführungsform einer erfindungsgemäßen Spritze 1 dargestellt, die einen zylinderförmigen Spritzenkörper 2 aufweist, der einen Innenraum 3 umfasst.

Der Innenraum 3 ist mit einer Spitze 4 verbunden und im Innenraum 3 ist eine Kolbenstange 5 längsverschieblich geführt.

Wie Fig. 1 verdeutlicht, ist auf der Spitze 4 ein Spritzenvorsatzfilter 6 fixiert. Das gesamte System ist ferner mit einem Verschlussstopfen 7 gasdicht verschlossen, der wiederum auf dem äußeren Ende des Spritzenvorsatzfilters 6 fixierbar ist.

Bei einer besonders bevorzugten Ausführungsform weist der Spritzenvorsatzfilter 6 einen ersten Fixierungsabschnitt 8 zur Anbringung an der Spitze 4 und einen zweiten Fixierungsabschnitt 9 zur Festlegung des Verschlussstopfens 7 auf.

Vorzugsweise sind der Spritzenvorsatzfilter 6 und der Verschlussstopfen 7 mittels jeweils einer Schraubverbindung 10 bzw. 11 fixierbar. Bei einer besonders bevorzugten Ausführungsform sind die Schraubverbindungen 10 und 11 jeweils als Luer-Lock-Verschluss ausgebildet.

Wie Fig. 1 ferner verdeutlicht, weist die Kolbenstange 5 an ihrem im Innenraum 3 angeordneten Endbereich 12 einen Gummistopfen 13 auf, der vorzugsweise siliconisiert ist.

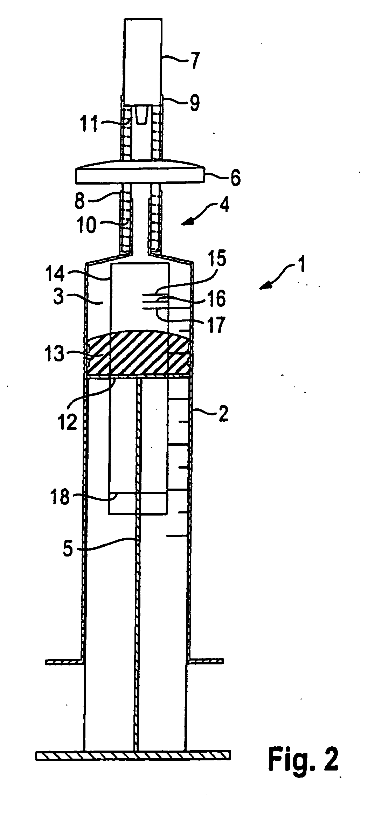

Die Ausführungsform gemäß Fig. 2 entspricht weitestgehend derjenigen der Fig. 1, so dass alle einander entsprechenden Merkmale mit denselben Bezugsziffern wie in Fig. 1 versehen sind. Insoweit kann auf die voranstehende Beschreibung der Fig. 1 verwiesen werden.

Die Ausführungsform der Spritze 1 gemäß Fig. 2 zeichnet sich dadurch aus, dass auf dem Spritzenkörper 2 ein Etikett 14 fixiert ist. Wie Fig. 2 verdeutlicht, weist das Etikett 14 Markierungen 15, 16 und 17 für unterschiedliche Mischungsverhältnisse zwischen schwerem Gas 19 und der anzusaugenden Luft auf. Ferner ist eine Markierung 18 für den maximalen Kolbenhub vorgesehen.

Wie eingangs bereits erläutert, kann die Spritze 1 als Einmalspritze ausgebildet sein.

Bevorzugterweise ist das im Innenraum 3 des Spritzenkörpers 2 eingebrachte Gas ein schweres Gas insbesondere SF₆, C₃F₈ oder C₂F₆.

Neben der voranstehenden schriftlichen Offenbarung der Erfindung wird hiermit explizit auf deren zeichnerische Darstellung in den Figuren 1 und 2 verwiesen.

### Bezugszeichenliste

- 1: Spritze
- 2: Spritzenkörper
- 3: Innenraum
- 4: Spitze
- 5: Kolbenstange
- 6: Spritzenvorsatzfilter
- 7: Verschlussstopfen
- 8: Fixierungsabschnitt
- 9: Fixierungsabschnitt
- 10, 11: Schraubverbindungen
- 13: Gummistopfen
- 14: Etikett
- 15, 16, 17: Markierung
- 19: Gas

## Patentansprüche

1. Spritze (1), in Form eines Ready to Use-Systems
- mit einem Spritzenkörper (2),
• der einen Innenraum (3) und
• der eine mit dem Innenraum (3) verbundene Spitze (4) aufweist;
- mit einer Kolbenstange (5), die längsverschieblich im Innenraum (3) geführt ist, und
- mit einem Verschlussstopfen (7),
**dadurch gekennzeichnet, dass** die spritze ein ready to use-system betrifft; wobei
- der Innenraum (3) als Gasbehälter ausgebildet und mit einem schweren Gas befüllt ist,
- ein Spritzenvorsatzfilter (6) auf der Spitze (4) fixiert ist, und
- der Verschlussstopfen (7) auf dem Spritzenvorsatzfilter (6) fixiert ist, der den Innenraum (3) gasdicht verschließt.

2. Spritze nach Anspruch 1, **dadurch gekennzeichnet, dass** der Spritzenvorsatzfilter (6) einen ersten Fixierungsabschnitt (8) zur Anbringung an der Spitze (4) und einen zweiten Fixierungsabschnitt (9) zur Festlegung des Verschlussstopfens (7) aufweist.

3. Spritze nach Anspruch 1 oder 2, **dadurch gekennzeichnet, dass** der Spritzenvorsatzfilter (6) und der Verschlussstopfen (7) mittels jeweils einer Schraubverbindung (10 bzw. 11) fixiert sind.

4. Spritze nach Anspruch 3, **dadurch gekennzeichnet, dass** die Schraubverbindung (10 bzw. 11) als Luer-Lock-Verschluss ausgebildet ist.

5. Spritze nach einem der Ansprüche 1 bis 4, **dadurch gekennzeichnet, dass** die Kolbenstange (5) an ihrem im Innenraum (3) angeordneten Endbereich (12) mit einem Gummistopfen (13) versehen ist.

6. Spritze nach Anspruch 5, **dadurch gekennzeichnet, dass** der Gummistopfen (13) siliconisiert ist.

7. Spritze nach einem der Ansprüche 1 bis 6, **dadurch gekennzeichnet, dass** auf dem Spritzenkörper (2) ein Etikett (14) mit Mischungsverhältnismarkierungen (15, 16 bzw. 17) und einer Markierung (18) für den maximalen Kolbenhub angebracht ist.

8. Spritze nach einem der Ansprüche 1 bis 7, **dadurch gekennzeichnet, dass** sie als Einmalspritze ausgebildet ist.

9. Spritze nach Anspruch 1, **dadurch gekennzeichnet, dass** das schwere Gas SF₆, C₃F₈ oder C₂F₆ ist.

## Claims

1. Syringe (1), in the form of a ready-to-use system
- having a syringe body (2),
• which has an interior space (3) and
• which has a tip (4) connected to the interior space (3);
- having a plunger rod (5) which is guided in the interior space (3) in a longitudinally displaceable manner, and
- having a sealing stopper (7),
**characterised in that** the syringe relates to a ready-to-use system; wherein
- the interior space (3) is formed as a gas container and filled with a heavy gas,
- a syringe attachment filter (6) is fixed on the tip (4), and
- the sealing stopper (7) is fixed on the syringe attachment filter (6), which closes the interior space (3) in a gas-tight manner.

2. Syringe according to Claim 1, **characterised in that** the syringe attachment filter (6) has a first fixing section (8) for mounting on the tip (4) and a second fixing section (9) for fastening the sealing stopper (7).

3. Syringe according to Claim 1 or 2, **characterised in that** the syringe attachment filter (6) and the sealing stopper (7) are fixed by means of a respective screw connection (10 and 11).

4. Syringe according to Claim 3, **characterised in that** the screw connection (10 and 11) is formed as a Luer-lock closure.

5. Syringe according to one of Claims 1 to 4, **characterised in that** the plunger rod (5) is provided with a rubber stopper (13) at its end region (12) that is arranged in the interior space (3).

6. Syringe according to Claim 5, **characterised in that** the rubber stopper (13) is silicone-treated.

7. Syringe according to one of Claims 1 to 6, **characterised in that** a label (14) with mixture-ratio marks (15, 16 and 17) and a mark (18) for the maximum plunger stroke is fixed on the syringe body (2).

8. Syringe according to one of Claims 1 to 7, **characterised in that** it is formed as a disposable syringe.

9. Syringe according to Claim 1, **characterised in that** the heavy gas is SF₆, C₃F₈ or C₂F₆.

## Revendications

1. Seringue (1) sous la forme d'un système prêt à l'emploi
- comprenant un corps de seringue (2)
• qui présente un espace interne (3) et
• qui présente une pointe (4) reliée à l'espace interne (3) ;
- comprenant un piston (5) qui est guidé dans l'espace interne (3) de manière à pouvoir coulisser en longueur et
- comprenant un bouchon de fermeture (7), **caractérisée en ce que** la seringue concerne un système prêt à l'emploi ; dans laquelle
- l'espace interne (3) est réalisé en tant que récipient de gaz et est rempli d'un gaz lourd,
- un filtre adaptateur de seringue (6) est fixé sur la pointe (4), et
- le bouchon de fermeture (7) qui ferme l'espace interne (3) de manière étanche aux gaz est fixé sur le filtre adaptateur de seringue (6).

2. Seringue selon la revendication 1, **caractérisée en ce que** le filtre adaptateur de seringue (6) présente une première section de fixation (8) pour le montage sur la pointe (4) et une seconde section de fixation (9) pour la fixation du bouchon de fermeture (7).

3. Seringue selon la revendication 1 ou 2, **caractérisée en ce que** le filtre adaptateur de seringue (6) et le bouchon de fermeture (7) sont fixés au moyen chacun d'une connexion vissée (10 ou 11).

4. Seringue selon la revendication 3, **caractérisée en ce que** la connexion vissée (10 ou 11) est réalisée en tant que fermeture Luer-Lock.

5. Seringue selon l'une quelconque des revendications 1 à 4, **caractérisée en ce que** le piston (5) est pourvu d'un bouchon en caoutchouc (13) au niveau de sa région d'extrémité (12) disposée dans l'espace interne (3).

6. Seringue selon la revendication 5, **caractérisée en ce que** le bouchon en caoutchouc (13) est siliconé.

7. Seringue selon l'une quelconque des revendications 1 à 6, **caractérisée en ce qu'**une étiquette (14) avec des marquages de rapport de mélange (15, 16 ou 17) et un marquage (18) pour la course de piston maximale est apposée sur le corps de seringue (2).

8. Seringue selon l'une quelconque des revendications 1 à 7, **caractérisée en ce qu'**elle est réalisée en tant que seringue à usage unique.

9. Seringue selon la revendication 1, **caractérisée en ce que** le gaz lourd est SF₆, C₃F₈ ou C₂F₆.
